# EUROPÄISCHE PATENTSCHRIFT

(11) **EP 1 788 975 B1**
(45) Veröffentlichungstag und Bekanntmachung des Hinweises auf die Patenterteilung: **10.12.2008**
(21) Anmeldenummer: 05779652.6
(22) Anmeldetag: 16.08.2005
(51) Int. Cl.: A61F 2/06

(54) **VERFAHREN ZUM ANBRINGEN EINER UMMANTELUNG AUF EINEM NATÜRLICHEN GEFÄSSEXPLANTAT MIT HILFE EINES ROHRFÖRMIGEN HILFSINSTRUMENTES**
METHOD FOR PLACING A JACKET ON A SECTION OF A NATURAL VESSELEXPLANT WITH THE AID OF A TUBULAR AUXILIARY INSTRUMENT
PROCEDE PERMETTANT D'APPLIQUER UNE GAINE SUR UNE SECTION VAISSEAU NATURELLE EXPLANTE A L'AIDE D'UN INSTRUMENT AUXILIAIRE TUBULAIRE

(30) Priorität: 19.08.2004 DE 102004041067
(43) Veröffentlichungstag der Anmeldung: 30.05.2007
(73) Patentinhaber: Aesculap AG, 78532 Tuttlingen (DE)
(72) Erfinder: BECK, Thomas, 78591 Durchhausen (DE); LANGANKE, Dennis, 78532 Tuttlingen (DE); MORITZ, Anton, 60590 Frankfurt/Main (DE)
(74) Vertreter: Patentanwälte Ruff, Wilhelm, Beier, Dauster & Partner
(86) Internationale Anmeldenummer: PCT/EP2005/008854
(87) Internationale Veröffentlichungsnummer: WO 2006/018268

(56) Entgegenhaltungen:
- DE-A1- 10 232 134
- US-B1- 6 620 176

## Beschreibung

Die Erfindung betrifft ein Verfahren zum Anbringen einer flexiblen Ummantelung auf ein natürliches Gefäßexplantat, insbesondere auf ein Venenexplantat und eine Vorrichtung zum Anbringen einer flexiblen Ummantelung gemäß diesem Verfahren.

Zur Behandlung von Gefäßerkrankungen durch Veränderungen an Blutgefässen wie beispielsweise Arteriosklerose werden in der modernen Chirurgie häufig sogenannte Bypässe in Form von Gefäßersatzimplantaten eingesetzt. Um unerwünschte Reaktionen und Wechselwirkungen wie beispielsweise Thrombusbildung durch nichtbiologische, wie z.B. metallische oder vollsynthetische Materialien, im Empfängerorganismus zu vermeiden, werden hierfür häufig biologische Gefäßmaterialien natürlichen Ursprungs eingesetzt. Diese natürlichen Gefäßersatzimplantate werden im allgemeinen aus Venen gewonnen. Damit sie den speziellen Anforderungen als beständige und stabile arterielle Ersatzgefäße, insbesondere als koronarer Bypass, entsprechen, müssen sie aufgrund des hohen arteriellen Gefäßinnendrucks in Ihrer Stabilität verstärkt und in ihrer äußeren Form angepasst werden.

Eine solche Verstärkung bzw. Anpassung kann beispielsweise durch Überziehen einer formgebenden bzw. stabilisierenden Ummantelung auf das Gefäß, insbesondere die Vene, erzielt werden. So wird gemäß der DE 199 10 340 ein Schlauch, ein Mantel oder eine Röhre als Ummantelung einer Vene für Arterienersatztransplantate verwendet. Ein Beispiel für eine rohrförmige Venenummantelung mit netzartiger Struktur findet sich in der DE 101 37 414 A1.

Um das Überziehen einer Ummantelung auf das natürliche Gefäß zu erleichtern und gleichzeitig die Gefahr einer Beschädigung der äußeren Gefäßwand zu vermeiden, kann die Ummantelung zunächst auf eine rohrförmige Applikationshilfe aufgezogen werden. Das Gefäß wird dann in die Applikationshilfe eingeführt, worauf diese unter gleichzeitigem Ablegen der Ummantelung auf der Oberfläche des Gefäßes wieder entfernt wird.

Ein grundlegendes Problem beim Anbringen der Ummantelung mittels einer rohrförmigen Applikationshilfe besteht jedoch darin, dass der unstabilisierte, extrem sensible, verletzungsempfindliche und äußerst flexible natürliche Gefäßabschnitt in die rohrförmige Applikationshilfe ein- und vollständig durch sie hindurch gezogen werden muss, ohne dass das Gefäß und insbesondere die Außenwände dadurch eine Beeinträchtigung oder Beschädigung erfahren. Zudem tritt beim Ablegen der Ummantelung auf das Gefäß häufig ein weiteres Problem auf. Bedingt durch die weiche, unstabilisierte Konsistenz, die ein natürliches Gefäß wie beispielsweise ein Venenexplantat aufweist, ist ein gleichmäßiges und insbesondere faltenfreies Ablegen der Ummantelung auf der Oberfläche des Gefäßes oft mit großen Schwierigkeiten verbunden. Natürlich darf auch der Gefäßabschnitt selbst in ummanteltem Zustand weder Knicke noch Falten aufweisen.

In Ann Thorac Surg 1994; 57; 240-2 wird das Einziehen einer Vene in eine rohrförmige Applikationshilfe mittels eines an ein Ende der Vene geknoteten Fadens beschrieben, welcher nach vollständigem Einziehen zusammen mit dem Ende der Vene abgeschnitten wird. Hierbei besteht allerdings, vor allem bei längeren Venenexplantaten, das Problem des Einführens des Fadens durch die Applikationshilfe.

In Ann Thorac Surg 1992; 127; 416-419 wird die Verwendung einer netzartigen, porösen und flexiblen Ummantelung zur Stabilisierung eines natürlichen, aus varikösen Venen gewonnenen, Venenexplantats sowie zur Korrektur und Anpassung seiner äußeren Form beschrieben. Zur generellen Untersuchung daraufhin, ob variköse Venen als Gefäßersatz geeignet sind, werden Venenabschnitte mit Röntgenkontrastmittel und anschließend mit Wachs befüllt und zur Auswertung in kurze Stücke geschnitten.

In US 4,743,251 wird in Figur 2B ein dünner Stab gezeigt, an welchen ein Venenende geknotet wird. Anschließend wird die befestigte Vene mittels des Stabes in und durch eine Applikationshilfe gezogen. Dann wird der Stab mit dem verknoteten Ende der Vene abgeschnitten.

Beide Vorgehensweisen, sowohl das Einziehen einer Vene in die Applikationshilfe mit einem Stab als auch mittels eines angeknoteten Fadens, weisen jedoch den entscheidenden Nachteil einer sehr hohen Belastung des Gefäßgewebes durch das extrem feine, reißfeste und dadurch sehr scharfe Fadenmaterial im Bereich des Knotens auf. Dies kann leicht zu einem Durchtrennen des Gefäßabschnitts, insbesondere im Falle variköser Venen, unmittelbar beim Festknoten und insbesondere innerhalb der Applikationshilfe während des Einziehens in die Ummantelung auf Grund der exakt im Punkt des Knotens auftretenden Zugkräfte führen. Zudem stellt das Befestigen oder Verknoten einen zusätzlichen, zeitaufwendigen Arbeitsschritt während der Operation dar.

In der DE 102 32 134 A1 der Anmelderin wird ein Hilfsinstrument zum Einziehen eines Gefäßabschnitts in eine Applikationshilfe beschrieben, das die Form eines stabförmigen Greifers aufweist. Durch den Einsatz eines solchen Greifers kommt man ohne weitere Befestigungsmaterialien aus und umgeht somit die oben im Zusammenhang mit dem Festknoten des Gefäßes beschriebenen Nachteile. Das Problem der Faltenbildung beim Ablegen der Ummantelung auf dem Gefäßabschnitt bleibt davon jedoch unberührt.

Es lässt sich festhalten, dass aus dem Stand der Technik für das Anbringen einer Ummantelung auf einen Gefäßabschnitt, insbesondere unter dem Aspekt der Faltenbildung beim Ablegen der Ummantelung auf den Gefäßabschnitt, noch keine umfassende und befriedigende Lösung bekannt ist.

Der Erfindung stellt sich daher die Aufgabe, ein Verfahren zum Anbringen einer flexiblen Ummantelung auf ein Gefäßexplantat bereitzustellen, welches die Probleme aus dem Stand der Technik überwindet. Insbesondere soll das Verfahren ein zuverlässiges, schnelles, einfaches und schonendes Einziehen des Gefäßes in eine Applikationshilfe in Verbindung mit einem faltenfreien Ablegen der Ummantelung auf der Oberfläche des Gefäßexplantats ermöglichen.

Diese Aufgabe wird gemäß der vorliegenden Erfindung gelöst durch ein Verfahren zum Anbringen einer flexiblen Ummantelung auf ein natürliches Gefäßexplantat, insbesondere auf ein Venenexplantat, mit den Schritten:
- Aufschieben der Ummantelung auf eine rohrförmige Applikationshilfe,
- Befüllen des Gefäßexplantats mit einem Fluid,
- Einbringen, insbesondere Einziehen, des befüllten Gefäßexplantats in die Applikationshilfe, wobei das Befüllen des Gefäßexplantats sowie das Einbringen des Gefäßexplantats in die Applikationshilfe mit Hilfe eines rohrförmigen Hilfsinstruments vorgenommen wird, wobei das Gefäßexplantat zum Befüllen und zum Einbringen in die Applikationshilfe mit dem ersten Ende des rohrförmigen Hilfsinstrumentes verbunden wird und das Befüllen mittels einer Befülleinrichtung erfolgt, die mit einer Anschlusseinrichtung des zweiten Endes des rohrförmigen Hilfsinstruments verbunden wird, und
- Auseinanderziehen von Applikationshilfe und rohrförmigem Hilfsinstrument unter gleichzeitigem Ziehen des Gefäßexplantats aus der Applikationshilfe und Ablegen der Ummantelung auf der Oberfläche des Gefäßexplantats.

Durch das Befüllen des Gefäßexplantats mit dem Fluid gelingt es, ein Gefäßexplantat mit weicher, unstabilisierter Konsistenz in eine mindestens teilstabilisierte Form, gegebenenfalls sogar gedehnte Form, mit einer vergleichsweise straffen und faltenfreien Oberfläche zu überführen. In dieser stabilisierten Form ist das Einbringen des befüllten Gefäßexplantats in die Applikationshilfe erleichtert, insbesondere aber in Bezug auf das faltenfreie Ablegen der Ummantelung auf der Oberfläche des befüllten Gefäßexplantats bieten sich gegenüber herkömmlichen Vorgehensweisen deutliche Vorteile.

Durch die Verwendung des rohrförmigen Hilfsinstruments sowohl beim Einziehen des Gefäßexplantats in die Applikationshilfe als auch beim Befüllen desselben wird eine einfache und schnelle Durchführbarkeit des Verfahrens gewährleistet. Der Füllgrad des Gefäßexplantats kann durch das rohrförmige Hilfsinstrument unmittelbar vor oder während des Einziehens des Gefäßexplantats eingestellt bzw. korrigiert werden. Die einzelnen Verfahrensschritte werden im folgenden noch detailliert beschrieben.

Das bevorzugt verwendete Hilfsinstrument lässt sich als ein gerades, vorzugsweise biegesteifes Rohr mit einem Durchgangskanal, vorzugsweise einem kontinuierlichen Durchgangskanal, beschreiben. Insbesondere wird ein Hilfsinstrument verwendet, das nach Art einer Kanüle ausgestaltet ist.

Das Gefäßexplantat wird zum Befüllen und zum Einbringen in die Applikationshilfe zunächst mit einem ersten Ende des rohrförmigen Hilfsinstruments verbunden. Besonders bevorzugt wird das Gefäßexplantat auf das Ende aufgesteckt. Das aufgesteckte Gefäßexplantat kann gegebenenfalls z.B. durch mechanische Mittel gegen Verrutschen bzw. Abrutschen von dem Ende des Hilfsinstruments gesichert, insbesondere geklammert oder festgebunden werden. Auch ein Ankleben des Gefäßexplantats ist möglich. Zum Festbinden kann übliches, dem Fachmann bekanntes, Nahtmaterial verwendet werden.

Das Befüllen des Gefäßexplantats wird mittels einer Befülleinrichtung, bevorzugt mittels einer Spritze, insbesondere einem Spritzenzylinder mit Schubkolben, vorgenommen. Dazu wird die Befülleinrichtung mit einem zweiten Ende des rohrförmigen Hilfsinstruments verbunden. Über das Hilfsinstrument kann somit ein an dessen erstem Ende befestigtes Gefäßexplantat mit dem Fluid befüllt werden. Mit besonderem Vorteil kann das Gefäßexplantat vor dem endgültigen Befüllen auch mit dem Fluid gespült werden. Auch eine Überprüfung auf Dichtigkeit ist möglich, was vor allem beim Vorhandensein einer oder mehrerer Ligaturen von großer Bedeutung sein kann. Hierzu wird die Vene an ihrem freien Ende verschlossen.

Als zweckmäßiges Fluid kommen grundsätzlich fließfähige Medien flüssiger und gasförmiger Art in Betracht. Allerdings werden zum Spülen und Befüllen vorzugsweise Flüssigkeiten, insbesondere wässrige Lösungen, z.B. physiologische Kochsalzlösung, eingesetzt.

In einer besonders bevorzugten Ausführungsform des erfindungsgemäßen Verfahrens wird zunächst die Ummantelung auf die rohrförmige Applikationshilfe aufgeschoben. Hierzu kann das Aufschiebende der rohrförmigen Applikationshilfe in bekannter Weise mit einem Stopfen mit einem Aufschiebekonus oder dergleichen versehen werden. Wie bereits beschrieben wird dann das zu ummantelnde Gefäßexplantat mit dem ersten Ende des rohrförmigen Hilfsinstruments verbunden. Danach wird das Hilfsinstrument vorzugsweise so weit durch die rohrförmige Applikationshilfe hindurchgeführt, dass das mit dem rohrförmigen Hilfsinstrument verbundene Gefäßexplantat noch nicht in die Applikationshilfe eingeführt ist, das zweite Ende des Hilfsinstruments aber schon aus dieser herausragt. Bevorzugt wird dann das zweite Ende des Hilfsinstruments zum Befüllen des Gefäßexplantats mit der Befülleinrichtung verbunden. Gleichermaßen kann es auch bevorzugt sein, dass das rohrförmige Hilfsinstrument zuerst durch die Applikationshilfe hindurchgeführt wird (bzw. sich gegebenenfalls bereits in dieser befindet) und dann sein erstes Ende mit dem Gefäßexplantat und sein zweites Ende mit der Befülleinrichtung verbunden wird. Vor dem Befüllen erfolgt gegebenenfalls ein Spülen des Gefäßexplantats mit dem Fluid sowie eine Prüfung auf Dichtigkeit. Vorzugsweise wird das Gefäßexplantat im nächsten Schritt über das rohrförmige Hilfsinstrument befüllt und anschließend durch Ziehen an dem Hilfsinstrument in die Applikationshilfe eingezogen. Alternativ können diese beiden Schritte aber auch in umgekehrter Reihenfolge vorgenommen werden. Abschließend werden die Applikationshilfe und das rohrförmige Hilfsinstrument auseinandergezogen, was unter gleichzeitigem Ziehen des Gefäßexplantats aus der Applikationshilfe erfolgt und unter Mitnahme und gleichzeitigem Ablegen der Ummantelung auf der Außenseite des Gefäßexplantats vorgenommen wird.

Als nach dem erfindungsgemäßen Verfahren zu ummantelnde, natürliche Gefäßexplantate werden Blutgefäßexplantate bevorzugt, vorzugsweise Teile von Venen, insbesondere von varikösen Venen. Mit einer Ummantelung versehene Venenexplantate können bis zu ihrer Verwendung in bekannter Weise gelagert werden.

Die vorliegende Erfindung umfasst auch eine Vorrichtung zum Anbringen einer flexiblen Ummantelung auf ein natürliches Gefäßexplantat gemäß einem Verfahren nach der Erfindung. Die erfindungsgemäße Vorrichtung umfasst ein rohrförmiges Hilfsinstrument mit einem Durchgangskanal, insbesondere nach Art einer Kanüle, eine rohrförmige Applikationshilfe, in die das rohrförmige Hilfsinstrument einführbar ist und eine flexible Ummantelung, die auf die rohrförmige Applikationshilfe aufschiebbar ist.

Die Applikationshilfe ist zweckmäßigerweise so lang, dass sie die Ummantelung, zumindest in zusammengeschobenem Zustand, vollständig aufzunehmen vermag.

Das rohrförmige Hilfsinstrument ist vorzugsweise länger als die Applikationshilfe. Ebenfalls ist es bevorzugt, dass das rohrförmige Hilfsinstrument über die gesamte Länge im Außendurchmesser kleiner als der Innendurchmesser der Applikationshilfe ist, so dass es zusammen mit dem Gefäßexplantat durch die Applikationshilfe hindurchführbar ist.

Die Applikationshilfe weist bevorzugt im wesentlichen die Form eines zylindrischen Rohrs auf. An ihrem einen Ende ist sie vorzugsweise trichterförmig aufgeweitet, um ein Einführen des Hilfsinstruments und des Gefäßexplantats auf dieser Seite zu erleichtern. Gegebenenfalls umfasst die Applikationshilfe einen konischen Stopfen zum Aufstecken an ihrem anderen Ende. Dieser ermöglicht ein erleichtertes, schonendes Aufbringen der Ummantelung auf die Applikationshilfe. Nach dem Aufbringen hat der konische Stopfen seinen Zweck erfüllt und kann wieder abgezogen werden. Grundsätzlich ist es auch möglich, den aufgesteckten Stopfen mit dem Hilfsinstrument beim Einführen desselben in die Applikationshilfe herauszustoßen.

Vorzugsweise ist die Innenoberfläche der Applikationshilfe glatt und frei von Unebenheiten, so dass ein verletzungsfreies Einziehen des Gefäßexplantats im Rahmen des erfindungsgemäßen Verfahrens gewährleistet ist.
Die Materialbeschaffenheit der Applikationshilfe ist grundsätzlich nicht kritisch. Gemäß einer Ausführungsform besteht mindestens ein Teil der Applikationshilfe aus metallischen Materialien, vorzugsweise aus rostfreiem federhartem Stahl. Es ist ebenfalls möglich, die Applikationshilfe aus nichtmetallischen Materialien, vorzugsweise aus Kunststoffen, insbesondere PPA, PEEK oder LCP (Liquid Crystal Polymer), herzustellen.

Die Ummantelung kann in einer besonderen Ausgestaltungsform der erfindungsgemäßen Vorrichtung bereits auf der Applikationshilfe angeordnet sein. Die Ummantelung ist vorzugsweise bereits in Röhrenform hergestellt, bevorzugt ist sie ausgebildet wie in der WO 03/011190 A2 beschrieben.

Entsprechend kann auch vorgesehen sein, dass das Hilfsinstrument bereits innerhalb der Applikationshilfe angeordnet ist. Es ist auch möglich, dass die Vorrichtung weitere Komponenten wie beispielsweise Nahtmaterial enthält, z.B. in Form eines Sets.

Es versteht sich für den Fachmann, dass die Applikationshilfe wie auch die übrigen Komponenten der Vorrichtung durch ein zweckmäßiges Sterilisationsverfahren nach üblichen chemischen oder physikalischen Methoden sterilisiert und insbesondere steril verpackt werden können.

Wie bereits erwähnt, ist es in Bezug auf die praktische Durchführbarkeit des beschriebenen erfindungsgemäßen Verfahrens von besonderem Vorteil, dass zwei so unterschiedliche Einzelschritte wie das Befüllen und das Einziehen des Gefäßexplantats unter Zuhilfenahme desselben Instruments durchgeführt werden können.

Nach der Erfindung weist das rohrförmiges Hilfsinstrument zur Verwendung in dem erfindungsgemäßen Verfahren einen Durchgangskanal auf, ist insbesondere nach Art einer Kanüle ausgestaltet. Es umfasst im wesentlichen einen rohrförmigen Abschnitt mit einem ersten und einem zweiten Ende. Das erste Ende des rohrförmigen Abschnitts ist mit Vorteil derart ausgestaltet, dass es einem aufgesteckten Gefäßexplantat einen Halt gegen Verrutschen oder Abrutschen ermöglicht. Eine Anschlusseinrichtung zur Befestigung einer Befülleinrichtung, insbesondere einer Spritze, ist an dem zweiten Ende des rohrförmigen Abschnitts vorgesehen.

Vorzugsweise weist das erste Ende des rohrförmigen Abschnitts ein Haltemittel auf, das insbesondere als mindestens eine ringförmige Verdickung ausgebildet ist. Bevorzugt verläuft diese quer zur Längsachse des rohrförmigen Abschnitts um dessen Außenumfang. Besonders bevorzugt ist das Haltemittel nach Art einer Olive ausgebildet. Nach außen hin kann das Ende konisch geformt sein, damit ein Venenexplantat leicht aufgeschoben werden kann. Alternativ kann es sich bei dem Haltemittel auch um eine Profilierung der Oberfläche des ersten Endes des rohrförmigen Abschnitts handeln.

In einer bevorzugten Ausführungsform des erfindungsgemäßen Hilfsinstruments ist die Anschlusseinrichtung zur Befestigung der Befülleinrichtung ein Adapter. Im Fall einer Spritze als Befülleinrichtung handelt es sich bei der Anschlusseinrichtung insbesondere um einen Luer-Lock-Adapter.

Es kann sich bei dem Hilfsinstrument um ein Einweg- oder um ein wiederverwendbares, resterilisierbares Produkt handeln. Bevorzugt ist das Hilfsinstrument aus den gleichen Materialien hergestellt, die auch für die Herstellung der Applikationshilfe in Frage kommen, also insbesondere aus metallischen Materialien wie z.B. rostfreiem, federhartem Stahl oder auch aus Kunststoff wie z.B. PPA, PEEK oder LCP.

Die vorliegende Erfindung umfasst auch die Verwendung der erfindungsgemäßen Vorrichtung zum Anbringen einer flexiblen Ummantelung auf ein natürliches Gefäßexplantat, insbesondere zum Einziehen des Gefäßexplantats in eine die Ummantelung tragende rohrförmige Applikationshilfe.

Im folgenden wird die vorliegende Erfindung und insbesondere ihre Funktionsweise durch ausführliche Beschreibung von besonderen Ausführungsformen erläutert. In diesen Ausführungsformen können einzelne Merkmale der Erfindung allein oder in Kombination mit anderen Merkmalen verwirklicht sein. Die beschriebenen besonderen Ausführungsformen dienen lediglich zur Erläuterung und zum besseren Verständnis der Erfindung und sind in keiner Weise einschränkend zu verstehen.

### Figurenbeschreibung

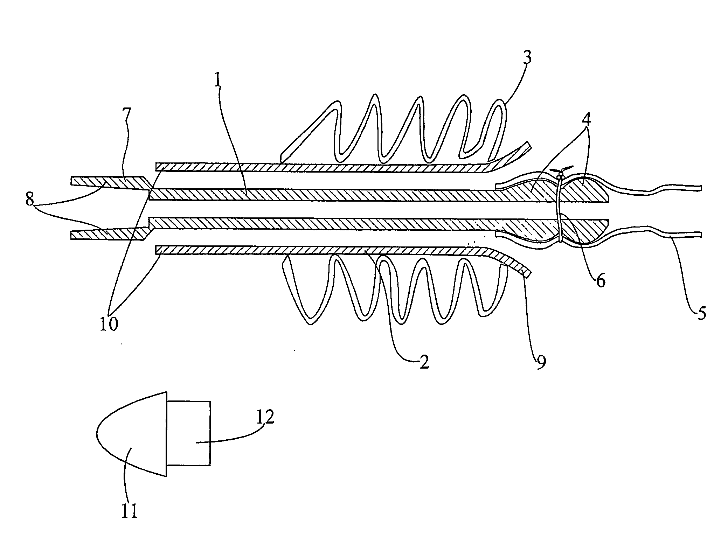

In der Zeichnung in **Fig. 1** ist ein Längsschnitt einer Ausführungsform der erfindungsgemäßen Vorrichtung zum Anbringen einer flexiblen Ummantelung auf ein Gefäßexplantat dargestellt. Eine Kanüle **1** als rohrförmiges Hilfsinstrument, das zum Einführen eines Gefäßexplantats, insbesondere einer Vene, in eine netzartige Ummantelung dient, ist umgeben von einer als Rohr **2** ausgebildeten Applikationshilfe. Auf ihrer Außenseite trägt das Rohr **2** die netzartige Ummantelung **3,** die zum Anbringen auf dem Gefäßexplantat vorgesehen ist. Die Ummantelung **3** ist schematisch gezeigt.

Das erste Ende der Kanüle **1** weist zwei ringförmige Verdickungen **4** auf, die als Haltemittel zur Befestigung des Gefäßexplantats **5** (ebenfalls schematisch gezeigt) dienen, der an dieses Ende der Kanüle ligiert ist. Im Bereich zwischen den zwei Verdickungen **4** ist das Gefäßexplantat **5** mit einem Faden **6** gegen Verrutschen bzw. Abrutschen von dem Ende gesichert. An das zweite Ende der Kanüle **1** ist ein Adapter **7** zum Anschließen einer Spritze angeformt. Der Adapter **7** besitzt einen Innenkonus **8,** in den ein Außenkonus eines Spritzenzylinders einsteckbar ist.

Das als Applikationshilfe dienende Rohr **2** weist im wesentlichen eine zylindrische Form mit einem kreisförmigen Querschnitt auf. Der Innendurchmesser des Rohres **2** ist gleich oder größer als der maximale Außendurchmesser der Kanüle **1** und gleich oder größer als der des durch das Rohr **2** zu ziehenden Gefäßexplantats **5.** An seinem einen Ende **9** ist das Rohr **2** trichterförmig aufgeweitet. An diesem Ende **9** ist die Kanüle **1** leicht einführbar. An seinem zweiten Ende **10** ist ein konisch ausgestalteter Stopfen **11** aufsteckbar. Bei aufgestecktem Stopfen **11** kann die Ummantelung **3** leicht auf das Rohr **2** aufgebracht werden. Der Stopfen ist mit einer Anschlagschulter **12** versehen, mit der er an der Stirnseite des Endes **10** zur Anlage kommt. Es können auch besondere Haltemittel vorgesehen sein.

Die Länge des Rohres **2** kann sich zwischen 4 cm und 20 cm bewegen, vorzugsweise zwischen 7 cm und 15 cm. Sein Durchmesser liegt bevorzugt zwischen 5 mm und 12 mm. Die Wandstärke des Rohres **2** beträgt vorteilhafterweise weniger als 0,5 mm, insbesondere 0,2 mm bis 0,3 mm. Das Rohr **2** hat einen bevorzugten Innendurchmesser zwischen 4,5 mm und 11,5 mm.

Die Kanüle **1** ist länger als das Rohr **2.** Ihre Länge liegt vorzugsweise zwischen 8 cm und 16 cm. Sie hat einen Außendurchmesser zwischen 4,5 mm und 10 mm. Der tatsächliche Wert ist jeweils abhängig vom Innendurchmesser des Rohres **2** (bzw. umgekehrt) und kann entlang der Kanüle **1** variieren. Vorzugsweise weist die Kanüle **1** im Bereich des Adapters **7** den größten Durchmesser auf. Der Durchmesser der Kanüle **1** ist im Bereich der Verdickungen **4** entsprechend bevorzugt kleiner. Der Innendurchmesser der Kanüle **1** sollte 1 mm nicht unterschreiten.

Zum Aufbringen einer netzartigen Umhüllung **3** auf ein Gefäßexplantat **5** mit der im Längsschnitt dargestellten Vorrichtung wird zunächst das zu ummantelnde Gefäßexplantat **5** auf das erste Ende der Kanüle **1** aufgesteckt. Dazu wird das Gefäßexplantat **5** über die zwei ringförmig ausgebildeten Verdickungen **4** geschoben. Gegebenenfalls wird das Gefäßexplantat **5** zwischen den beiden Verdickungen **4** mit einem Faden **6** zusätzlich vor dem Abrutschen gesichert. Danach wird eine Spritze an den Adapter **7** angeschlossen. Das Gefäßexplantat **5** kann dann z.B. mit physiologischer Kochsalzlösung gespült werden. Auch die Dichtigkeit von eventuell vorhandenen Seitenastligaturen kann überprüft werden, indem das Gefäßexplantat **5** durch Einspritzen der Lösung unter Druck gedehnt wird. Dazu wird das Gefäßexplantat **5** an seinem freien Ende verschlossen. Falls noch nicht geschehen, wird der konisch ausgestaltete Stopfen **11** auf das zweite Ende **10** der Rohres **2** gesteckt. Die Ummantelung **3** wird auf das Rohr **2** aufgeschoben, worauf der Stopfen **11** wieder entfernt werden kann. Zum Einziehen des Gefäßexplantats **5** in das Rohr **2** wird eine gegebenenfalls angeschlossene Spritze vom Adapter **7** abgezogen und die Kanüle **1** mit dem Adapter **7** voran durch das trichterförmig aufgeweitete Ende **9** durch das Rohr **2** geschoben, bis der Adapter **7** am anderen Ende **10** des Rohres **2** greifbar ist. Die Spritze kann nun wieder angeschlossen werden. Es ist auch möglich, die Spritze als Handgriff für die aufgesteckte Kanüle 1 zu benutzen. Auf diese Weise kann die Kanüle vom zweiten Ende **10** des Rohres in dieses eingeschoben werden, nachdem die Ummantelung bereits auf das Rohr **2** aufgeschoben ist. Erst dann wird das Gefäßexplantat **5** am ersten Ende bzw. den Verdickungen **4** der Kanüle **1** befestigt und gegebenenfalls nach Spülen befüllt. Nun kann das Gefäßexplantat **5** durch Ziehen an der Kanüle in das als Applikationshilfe dienende Rohr **2** gezogen und anschließend aus dieser herausgezogen werden. Beim Herausziehen wird die Ummantelung **3** gleichmäßig auf der Außenseite des Gefäßexplantats **5** abgelegt. Mit angeschlossener Spritze kann über die Kanüle **1** unmittelbar vor oder während des Einziehens der Füllgrad des Gefäßexplantats **5** eingestellt bzw. korrigiert werden. Wenn die Ummantelung **3** aufgebracht ist, kann das ummantelte Gefäßexplantat **5** vom Ende der Kanüle **1** abgezogen bzw. abgeschnitten werden und auf die benötigte Länge zugeschnitten werden.

## Patentansprüche

1. Verfahren zum Anbringen einer flexiblen Ummantelung **(3)** auf ein natürliches Gefäßexplantat **(5),** insbesondere auf einem Venenexplantat, umfassend die Schritte
- Aufschieben der Ummantelung **(3)** auf eine rohrförmige Applikationshilfe **(2),**
- Befüllen des Gefäßexplantats **(5)** mit einem Fluid,
- Einbringen, insbesondere Einziehen, des befüllten Gefäßexplantats **(5)** in die Applikationshilfe **(2),** wobei das Befüllen des Gefäßexplantats **(5)** sowie das Einbringen des Gefäßexplantats **(5)** in die Applikationshilfe **(2)** mit Hilfe eines rohrförmigen Hilfsinstruments **(1)** vorgenommen wird, wobei das Gefäßexplantat **(5)** zum Befüllen und zum Einbringen in die Applikationshilfe **(2)** mit dem ersten Ende des rohrförmigen Hilfsinstruments **(1)** verbunden wird und das Befüllen mittels einer Befülleinrichtung erfolgt, die mit einer Anschlusseinrichtung **(7)** des zweiten Endes des rohrförmigen Hilfsinstruments **(1)** verbunden wird, und
- Auseinanderziehen von Applikationshilfe **(2)** und rohrförmigem Hilfsinstrument **(1)** unter gleichzeitigem Ziehen des Gefäßexplantats **(5)** aus der Applikationshilfe **(2)** und Ablegen der Ummantelung **(3)** auf der Oberfläche des Gefäßexplantats **(5).**

2. Verfahren nach Anspruch 1, **dadurch gekennzeichnet, dass** als Hilfsinstrument ein Rohr **(1)** mit einem Durchgangskanal, insbesondere nach Art einer Kanüle, verwendet wird.

3. Verfahren nach einem der Ansprüche 1 oder 2, **dadurch gekennzeichnet, dass** das Gefäßexplantat **(5)** zum Befüllen und zum Einbringen in die Applikationshilfe **(2)** auf das erste Ende des rohrförmigen Hilfsinstruments **(1)** aufgesteckt wird.

4. Verfahren nach einem der Ansprüche 1 bis 3, **dadurch gekennzeichnet, dass** das Befüllen mittels einer Spritze erfolgt, die mit dem zweiten Ende des rohrförmigen Hilfsinstruments **(1)** verbunden wird.

5. Verfahren nach einem der vorhergehenden Ansprüche, **dadurch gekennzeichnet, dass** als Fluid eine Flüssigkeit, insbesondere eine wässrige Lösung, verwendet wird.

6. Verfahren nach einem der vorhergehenden Ansprüche, umfassend den schritt,
- gegebenenfalls Spülen des Gefäßexplantats **(5)** mit dem Fluid.

7. Vorrichtung zum Anbringen einer flexiblen Ummantelung **(3)** auf ein natürliches Gefäßexplantat **(5)** gemäß dem Verfahren nach einem der vorhergehenden Ansprüche, umfassend ein rohrförmiges Hilfsinstrument **(1)** mit einem Durchgangskanal, insbesondere nach Art einer Kanüle **(1),** eine rohrförmige Applikationshilfe **(2),** in die das rohrförmige Hilfsinstrument **(1)** einführbar ist und eine flexible Ummantelung **(3),** die auf die rohrförmige Applikationshilfe **(2)** aufschiebbar ist, wobei das rohrförmige Hilfsinstrument (1) einen rohrförmigen Abschnitt mit einem ersten und einem zweiten Ende umfasst, wobei das erste Ende des rohrförmigen Abschnitts derart ausgestaltet ist, dass es einem auf das erste Ende aufgesteckten Gefäßexplantat **(5)** einen Halt gegen Verrutschen oder Abrutschen ermöglicht, **dadurch gekennzeichnet, dass** das zweite Ende eine Anschlusseinrichtung **(7)** zur Befestigung einer Befülleinrichtung, insbesondere einer Spritze, umfasst.

8. Vorrichtung nach Anspruch 7, **dadurch gekennzeichnet, dass** die Applikationshilfe **(2)** so lang ist, dass sie die Ummantelung **(3),** gegebenenfalls in zusammengeschobenem Zustand, vollständig aufzunehmen vermag.

9. Vorrichtung nach einem der Ansprüche 7 oder 8, **dadurch gekennzeichnet, dass** das rohrförmige Hilfsinstrument **(1)** länger ist als die Applikationshilfe **(2).**

10. Vorrichtung nach einem der Ansprüche 7 bis 9, **dadurch gekennzeichnet, dass** sich die Ummantelung **(3)** auf der Applikationshilfe **(2)** befindet.

11. Vorrichtung nach einem der Ansprüche 7 bis 10, **dadurch gekennzeichnet, dass** das rohrförmige Hilfsinstrument **(1)** durch die Applikationshilfe **(2)** hindurchführbar ist.

12. Vorrichtung nach einem der Ansprüche 7 bis 11, **dadurch gekennzeichnet, dass** das erste Ende mindestens ein Haltemittel aufweist, das insbesondere als ringförmige Verdickung **(4)** ausgebildet ist, die vorzugsweise quer zur Längsachse des rohrförmigen Abschnitts **(1)** um dessen Außenumfang verläuft.

13. Vorrichtung nach einem der Ansprüche 7 bis 12, **dadurch gekennzeichnet, dass** die Anschlusseinrichtung **(7)** ein Adapter zur Befestigung der Befülleinrichtung ist.

## Claims

1. Method for placing a flexible jacket (3) on a natural vessel explant (5), in particular a vein explant, said method comprising the following steps:
- pushing the jacket (3) onto a tubular application aid (2),
- filling the vessel explant (5) with a fluid,
- introducing, in particular pulling, the filled vessel explant (5) into the application aid (2), the filling of the vessel explant (5) and the introduction of the vessel explant (5) into the application aid (2) being carried out with the assistance of a tubular auxiliary instrument (1), the vessel explant (5) to be filled and to be introduced into the application aid (2) being connected to the first end of the tubular auxiliary instrument (1) and the filling being carried out by means of a filling device, which is connected to a connector piece (7) of the second end of the tubular auxiliary instrument (1), and
- pulling the application aid (2) and the tubular auxiliary instrument (1) apart while at the same time pulling the vessel explant (5) out of the application aid (2) and depositing the jacket (3) on the surface of the vessel explant (5).

2. Method according to Claim 1, **characterized in that** a tube (1) with a through-channel, in particular in the manner of a cannula, is used as auxiliary instrument.

3. Method according to either of Claims 1 and 2, **characterized in that** the vessel explant (5) to be filled and to be introduced into the application aid (2) is fitted onto the first end of the tubular auxiliary instrument (1).

4. Method according to one of Claims 1 to 3, **characterized in that** the filling is carried out by means of a syringe, which is connected to the second end of the tubular auxiliary instrument (1).

5. Method according to one of the preceding claims, **characterized in that** a liquid, in particular an aqueous solution, is used as fluid.

6. Method according to one of the preceding claims, comprising the following step:
- optionally flushing the vessel explant (5) with the fluid.

7. Device for placing a flexible jacket (3) on a natural vessel explant (5) in accordance with the method according to one of the preceding claims, said device comprising a tubular auxiliary instrument (1) with a through-channel, in particular in the manner of a cannula (1), a tubular application aid (2) into which the tubular auxiliary instrument (1) can be inserted, and a flexible jacket (3) that can be pushed onto the tubular application aid (2), the tubular auxiliary instrument (1) comprising a tubular portion with a first end and a second end, the first end of the tubular portion being configured in such a way that it provides a vessel explant (5) fitted onto the first end with a secure hold against sliding out of place or slipping off, **characterized in that** the second end comprises a connector piece (7) for securing a filling device, in particular a syringe.

8. Device according to Claim 7, **characterized in that** the application aid (2) is of such length that it is able to completely receive the jacket (3), if appropriate in the contracted state.

9. Device according to either of Claims 7 and 8, **characterized in that** the tubular auxiliary instrument (1) is longer than the application aid (2).

10. Device according to one of Claims 7 to 9, **characterized in that** the jacket (3) is located on the application aid (2).

11. Device according to one of Claims 7 to 10, **characterized in that** the tubular auxiliary instrument (1) can be guided through the application aid (2).

12. Device according to one of Claims 7 to 11, **characterized in that** the first end has at least one retaining means designed in particular as an annular enlargement (4) that preferably extends transverse to the longitudinal axis of the tubular portion (1), around the outer circumference of the latter.

13. Device according to one of Claims 7 to 12, **characterized in that** the connector piece (7) is an adapter for securing the filling device.

## Revendications

1. Procédé d'application d'une gaine flexible (3) sur un explant vasculaire naturel (5), en particulier sur un explant veineux, lequel procédé comporte les étapes qui consistent à :
- enfiler la gaine (3) sur un accessoire tubulaire d'application (2),
- remplir l'explant vasculaire (5) d'un fluide,
- insérer, en particulier en le tirant, l'explant vasculaire (5) rempli dans l'accessoire d'application (2), le remplissage de l'explant vasculaire (5) ainsi que l'insertion de l'explant vasculaire (5) dans l'accessoire d'application (2) étant réalisés à l'aide d'un instrument auxiliaire (1) de forme tubulaire, l'explant vasculaire (5) étant relié à la première extrémité de l'instrument auxiliaire (1) de forme tubulaire pour être rempli et inséré dans l'accessoire d'application (2) et le remplissage s'effectuant à l'aide d'un dispositif de remplissage relié à un dispositif de raccordement (7) de la deuxième extrémité de l'instrument auxiliaire (1) de forme tubulaire et
- écarter l'un de l'autre l'accessoire d'application (2) et l'instrument auxiliaire (1) de forme tubulaire tout en tirant l'explant vasculaire (5) hors de l'accessoire d'application (2) et en déposant la gaine (3) à la surface de l'explant vasculaire (5).

2. Procédé selon la revendication 1, **caractérisé en ce que** comme l'instrument auxiliaire, on utilise un tube (1) traversé par un canal, en particulier de type canule.

3. Procédé selon l'une des revendications 1 ou 2, **caractérisé en ce que** pour être rempli et inséré dans l'accessoire d'application (2), l'explant vasculaire (5) est enfiché sur la première extrémité de l'instrument auxiliaire (1) de forme tubulaire.

4. Procédé selon l'une des revendications 1 à 3, **caractérisé en ce que** le remplissage s'effectue au moyen d'une seringue reliée à la deuxième extrémité de l'instrument auxiliaire (1) de forme tubulaire.

5. Procédé selon l'une des revendications précédentes, **caractérisé en ce que** comme fluide, on utilise un liquide et en particulier une solution aqueuse.

6. Procédé selon l'une des revendications précédentes, qui comprend l'étape qui consiste à éventuellement rincer l'explant vasculaire (5) avec le fluide.

7. Dispositif d'application d'une gaine flexible (3) sur un explant vasculaire naturel (5) par le procédé selon l'une des revendications précédentes, lequel dispositif comprend un instrument auxiliaire (1) de forme tubulaire traversé par un canal, en particulier du type canule (1), un accessoire tubulaire d'application (2) dans lequel l'instrument auxiliaire (1) de forme tubulaire peut être inséré et une gaine flexible (3) qui peut être glissée sur l'accessoire tubulaire d'application (2), l'instrument auxiliaire (1) de forme tubulaire comprenant une partie tubulaire dotée d'une première et d'une deuxième extrémité, la première extrémité de la partie tubulaire étant configurée de manière à empêcher l'explant vasculaire (5) enfiché sur la première extrémité de glisser ou de se détacher,
**caractérisé en ce que**
la deuxième extrémité comprend un dispositif de raccordement (7) destiné à fixer un dispositif de remplissage, en particulier une seringue.

8. Dispositif selon la revendication 7, **caractérisé en ce que** l'auxiliaire d'application (2) est suffisamment long pour pouvoir reprendre complètement la gaine (3), éventuellement à l'état comprimé.

9. Dispositif selon l'une des revendications 7 ou 8, **caractérisé en ce que** l'instrument auxiliaire (1) de forme tubulaire est plus long que l'accessoire d'application (2).

10. Dispositif selon l'une des revendications 7 à 9, **caractérisé en ce que** la gaine (3) est située sur l'accessoire d'application (2).

11. Dispositif selon l'une des revendications 7 à 10, **caractérisé en ce que** l'instrument auxiliaire (1) de forme tubulaire peut être passé à travers l'accessoire d'application (2).

12. Dispositif selon l'une des revendications 7 à 11, **caractérisé en ce que** la première extrémité présente au moins un moyen de retenue qui est configuré en particulier comme épaississement annulaire (4) et qui s'étend de préférence à la périphérie de la partie tubulaire (1), transversalement par rapport à son axe longitudinal.

13. Dispositif selon l'une des revendications 7 à 12, **caractérisé en ce que** le dispositif de raccordement (7) est un adaptateur de fixation du dispositif de remplissage.
